# EUROPEAN PATENT SPECIFICATION

(11) **EP 2 417 106 B1**
(45) Date of publication and mention of the grant of the patent: **30.11.2016**
(21) Application number: 10713193.0
(22) Date of filing: 07.04.2010
(51) Int. Cl.: C07D 207/12, C07D 233/60, A61K 9/14, A61K 31/40

(54) **PROCESS FOR PREPARING PYRROLIDINIUM SALTS**
VERFAHREN ZUR HERSTELLUNG VON PYRROLIDINIUMSALZEN
PROCÉDÉ DE PRÉPARATION DE SELS DE PYRROLIDINIUM

(30) Priority: 09.04.2009 US 167977 P
(43) Date of publication of application: 15.02.2012
(73) Proprietor: Novartis AG, 4056 Basel (CH)
(72) Inventor: ALLMENDINGER, Thomas, CH-4002 Basel (CH)
(74) Representative: Frigola Deulofeu, Maria Carmen
(86) International application number: PCT/EP2010/054610
(87) International publication number: WO 2010/115937

(56) References cited:
- WO-A-03/087094
- WO-A-2004/054971
- WO-A1-2005/090342
- WO-A1-2007/068443
- GB-A- 1 350 928
- US-A1- 2002 173 536

## Description

### Field of the invention

This invention relates to pyrrolidinium compounds and their use as pharmaceuticals, in particular an industrial scale process for preparing glycopyrronium bromide and analogues.

### Background

Glycopyrronium bromide, also known as 3-[(cyclopentylhydroxyphenylacetyl)oxy]-1,1-dimethylpyrrolidinium bromide or glycopyrrolate, is an antimuscarinic agent that is currently administered by injection to reduce secretions during anaesthesia and or taken orally to treat gastric ulcers.

It has the following chemical structure:

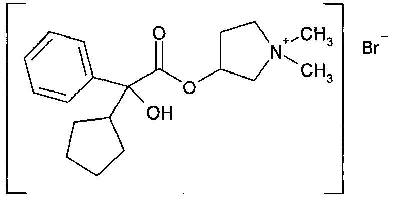

United States patent US 2,956,062 discloses that 1-methyl-3-pyrrolidyl alpha-cyclopentyl mandelate and can be prepared from methyl alpha cyclopentylmandelate and that the methyl bromide quaternary salt can be prepared by saturating a solution of 1-methyl-3-pyrrolidyl alpha-cyclopentyl mandelate in dry ethyl acetate with methyl bromide and filtering the crystalline solid that appears on standing.

The process of US 2,956,062 for preparing 1-methyl-3-pyrrolidyl alpha-cyclopentyl mandelate involves transesterifying methyl glycolate with an amino alcohol under the influence of metallic sodium to give a glycolate intermediate. Metallic sodium is highly reactive, which poses health and safety risks that make its use undesirable on an industrial scale for commercial manufacture.

The process of US 2,956,062 requires preparing the methylester in a previous step and alkylating the amino esters in a later step to form the desired quaternary ammonium salts.

The process of US 2,956,062 provides a mixture of diastereoisomers. The relative proportions of the diastereoisomers can vary widely between batches. This variation can give rise to surprising differences when preparing dry powder formulations from glycopyrronium bromide, which can cause problems when formulating such dry powders for pharmaceutical use.

United States patent application US 2007/0123557 discloses 1-(alkoxycarbonylmethyl)-1-methylpyrrolidyl anticholinergic esters. It describes coupling (R)-cyclopentylmandelic acid with (R,S)-1-methyl-pyrrolidin-3-ol under Mitsunobu conditions to give pure (R)-stereoisomeric compounds that are reacted with a bromoacetate to give the desired esters. It should be noted however that the chemicals used in Mitsunobu reactions, typically dialkyl azodicarboxylates and triphenylphosphine, pose health, safety and ecological risks that make their use undesirable on an industrial scale for commercial manufacture. They are also generally too expensive to source and too laborious to use in commercial manufacture.

United States patent application US 2006/0167275 discloses a process for the enrichment of the R, R- or S, S-configured glycopyrronium isomers and their thienyl analogues having R, S or S, R configuration.

WO 03/087094 A2 discloses new therapeutically useful pyrrolidinium derivatives, processes for their preparation and pharmaceutical compositions containing them.

There is therefore a need to provide a process for preparing glycopyrronium bromide that addresses the aforementioned problems identified in the known process or at least provides a useful alternative to it.

### Statement of the invention

In a first aspect, a process for preparing a compound of formula I 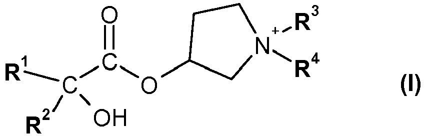 in salt or zwitterionic form, wherein
R¹ and R² are each independently C₃-C₈-cycloalkyl or C₆-C₁₀-aryl; and
R³ and R⁴ are each independently C₁-C₈-alkyl;
the process comprising the steps of:
(a) reacting a compound of formula II 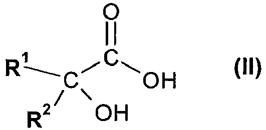 or a salt thereof wherein R¹ and R² are each independently C₃-C₈-cycloalkyl or C₆-C₁₀-aryl, with a compound of formula III 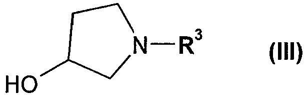 or an ester-forming derivative thereof, wherein R³ is C₁-C₈-alkyl to form a compound of formula IV 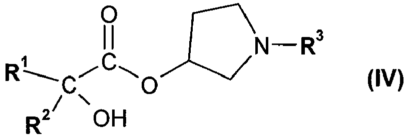 wherein R¹ and R² are each independently C₃-C₈-cycloalkyl or C₆-C₁₀-aryl and R³ is C₁-C₈-alkyl; and
(b) reacting a compound of formula IV wherein R¹ and R² are each independently C₃-C₈-cycloalkyl or C₆-C₁₀-aryl and R³ is C₁-C₈-alkyl with a compound of formula V

   X-R⁴ (V)

   wherein R⁴ is C₁-C₈-alkyl and X is a leaving group, to form a compound of formula I in salt or zwitterionic form, wherein
   R¹ and R² are each independently C₃-C₈-cycloalkyl or C₆-C₁₀-aryl; and
   R³ and R⁴ are each independently C₁-C₈-alkyl.

Step (a) is suitably carried out in the presence of a coupling agent, for example carbonyldiimidazole.

In a second aspect, the present invention provides a process for preparing an inhalable dry powder formulation of a compound of formula I 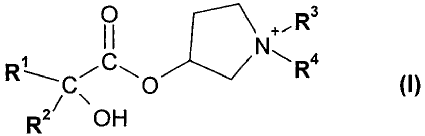 in salt or zwitterionic form, wherein
R¹ and R² are each independently C₃-C₈-cycloalkyl or C₆-C₁₀-aryl; and
R³ and R⁴ are each independently C₁-C₈-alkyl;
the process comprising the steps of:
(i) reacting a compound of formula II 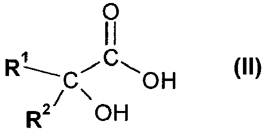 or a salt thereof wherein R¹ and R² are each independently C₃-C₈-cycloalkyl or C₆-C₁₀-aryl, with a compound of formula III 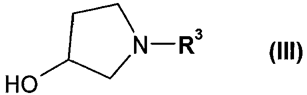 or an ester-forming derivative thereof, wherein R³ is C₁-C₈-alkyl to form a compound of formula IV 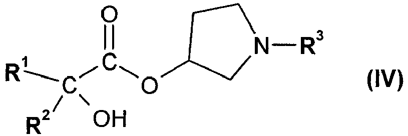 wherein R¹ and R² are each independently C₃-C₈-cycloalkyl or C₆-C₁₀-aryl and R³ is C₁-C₈-alkyl;
(ii) reacting a compound of formula IV wherein R¹ and R² are each independently C₃-C₈-cycloalkyl or C₆-C₁₀-aryl and R³ is C₁-C₈-alkyl with a compound of formula V

   X-R⁴ (V)

   wherein R⁴ is C₁-C₈-alkyl and X is a leaving group, to form a drug substance that comprises a compound of formula I in salt or zwitterionic form, wherein R¹ and R² are each independently C₃-C₈-cycloalkyl or C₆-C₁₀-aryl; and
   R³ and R⁴ are each independently C₁-C₈-alkyl;
(iii) optionally purifying the drug substance by crystallisation to provide a purified drug substance;
(iv) micronising the drug substance; and
(v) admixing carrier particles to give the inhalable dry powder.

In a preferred embodiment the carrier particles are crystalline sugars, especially lactose monohydrate or anhydrous lactose.

In a preferred embodiment the crystalline glycopyrrolate is micronised together with a force control agent. The force control agent is preferably magnesium stearate.

### Terms

Terms used in the specification have the following meanings:

"C₁-C₈-alkyl" as used herein denotes straight chain or branched C₁-C₈-alkyl having 1 to 8 carbon atoms, which may be, for example, methyl, ethyl, n-propyl, isopropyl, n-butyl, isobutyl, sec-butyl, tert-butyl, straight or branched pentyl, straight or branched hexyl, straight or branched heptyl, or straight or branched octyl. "C₁-C₈-alkyl" is suitably C₁-C₄-alkyl, especially methyl.

"C₃-C₈-cycloalkyl" as used herein denotes cycloalkyl having 3 to 8 carbon atoms, which may be, for example, cyclopropyl, cyclobutyl, cyclopentyl, methylcyclopentyl, cyclohexyl, methylcyclohexyl, dimethylcyclohexyl, cycloheptyl, bicycloheptyl, cyclooctyl and bicyclooctyl. "C₃-C₈-cycloalkyl" is suitably "C₃-C₆-cycloalkyl", especially cyclopropyl.

"C₆-C₁₀-aryl" as used herein denotes an aromatic group having 6- to 10-ring carbon atoms. Examples of C₆-C₁₀-aryl groups include but are not limited to phenyl, indanyl, indenyl and naphthyl. "C₆-C₁₀-aryl" is suitably phenyl.

"Leaving group" as used herein denotes a chemical group that departs with a pair of electrons in heterolytic bond cleavage. It is well known in the art that leaving groups can take many forms the term is therefore is intended to encompass any chemical group that fulfils the aforementioned function. Leaving groups can be anions or neutral molecules. Common anionic leaving groups are halides such as Cl⁻, Br⁻, and I⁻, and sulfonate esters esters, such as *para*-toluenesulfonate or "tosylate" (TsO⁻). Common neutral molecule leaving groups are water, ammonia, and alcohols. In the process of the present invention the leaving group is an anionic leaving group, for example Cl⁻, Br⁻ or I⁻, especially Br⁻.

"Salt" as used herein refers to an acid addition or base addition salt of a compound of the invention. "Salts" include in particular "pharmaceutical acceptable salts". The term "pharmaceutically acceptable salts" refers to salts that retain the biological effectiveness and properties of the compounds of this invention and, which typically are not biologically or otherwise undesirable. In many cases, the compounds of the present invention are capable of forming acid and/or base salts by virtue of the presence of amino and/or carboxyl groups or groups similar thereto. Pharmaceutically acceptable acid addition salts can be formed with inorganic acids and organic acids, e.g., acetate, aspartate, benzoate, besylate, bromide/hydrobromide, bicarbonate/carbonate, bisulfate/sulfate, camphorsulfornate, chloride/hydrochloride, chlortheophyllonate, citrate, ethandisulfonate, fumarate, gluceptate, gluconate, glucuronate, hippurate, , hydroiodide/iodide, isethionate, lactate, lactobionate, laurylsulfate, malate, maleate, malonate, mandelate, mesylate, methylsulphate, naphthoate, napsylate, nicotinate, nitrate, octadecanoate, oleate, oxalate, palmitate, pamoate, phosphate/hydrogen phosphate/dihydrogen phosphate, polygalacturonate, propionate, stearate, succinate, sulfosalicylate, tartrate, tosylate and trifluoroacetate salts. Inorganic acids from which salts can be derived include, for example, hydrochloric acid, hydrobromic acid, sulfuric acid, nitric acid, phosphoric acid, and the like. Organic acids from which salts can be derived include, for example, acetic acid, propionic acid, glycolic acid, oxalic acid, maleic acid, malonic acid, succinic acid, fumaric acid, tartaric acid, citric acid, benzoic acid, mandelic acid, methanesulfonic acid, ethanesulfonic acid, toluenesulfonic acid, sulfosalicylic acid, and the like. Pharmaceutically acceptable base addition salts can be formed with inorganic and organic bases. Inorganic bases from which salts can be derived include, for example, ammonium salts and metals from columns I to XII of the periodic table. In certain embodiments, the salts are derived from sodium, potassium, ammonium, calcium, magnesium, iron, silver, zinc, and copper; particularly suitable salts include ammonium, potassium, sodium, calcium and magnesium salts. Organic bases from which salts can be derived include, for example, primary, secondary, and tertiary amines, substituted amines including naturally occurring substituted amines, cyclic amines, basic ion exchange resins, and the like. Certain organic amines include isopropylamine, benzathine, cholinate, diethanolamine, diethylamine, lysine, meglumine, piperazine and tromethamine. The pharmaceutically acceptable salts of the present invention can be synthesized from a parent compound, a basic or acidic moiety, by conventional chemical methods. Generally, such salts can be prepared by reacting free acid forms of these compounds with a stoichiometric amount of the appropriate base (such as Na, Ca, Mg, or K hydroxide, carbonate, bicarbonate or the like), or by reacting free base forms of these compounds with a stoichiometric amount of the appropriate acid. Such reactions are typically carried out in water or in an organic solvent, or in a mixture of the two. Generally, use of non-aqueous media like ether, ethyl acetate, ethanol, isopropanol, or acetonitrile is desirable, where practicable. The compounds of formula I are most suitably bromide salts.

"Zwitterionic" as used herein refers to internal salts that bare formed when both a basic group and an acid group are present in the same molecule. For example, compounds of formula I contain an acidic carboxyl group that can exist as zwitterions with the quaternary ammonium atom.

Throughout this specification and in the claims that follow, unless the context requires otherwise, the word "comprise", or variations such as "comprises" or "comprising", will be understood to imply the inclusion of a stated integer or step or group of integers or steps but not the exclusion of any other integer or step or group of integers or steps.

### Detailed description

The present invention provides a process for preparing compounds of formula I 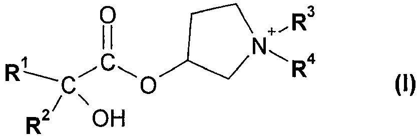 in salt or zwitterionic form, wherein
R¹ and R² are each independently C₃-C₈-cycloalkyl or C₆-C₁₀-aryl; and
R³ and R⁴ are each independently C₁-C₈-alkyl.

A most preferred compound of formula I is glycopyrronium bromide or glycopyrrolate that has the following chemical structure:

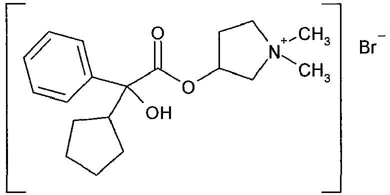

Glycopyrronium bromide has two stereogenic centres and hence exists in four isomeric forms or stereoisomers, namely (3R,2'R)-, (3S,2'R)-, (3R,2'S)- and (3S,2'S)-3-[(cyclopentyl-hydroxyphenylacetyl)-oxy]-1,1-dimethylpyrrolidinium bromide.

The process is a two-step process for preparing compounds of formula I, especially glycopyrronium bromide, that may be carried out in a single reaction vessel i.e. a one-pot process.

In the first step (a) of the process of the invention, a compound of formula II 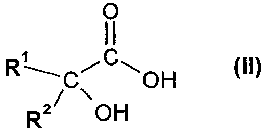 or a salt thereof wherein R¹ and R² are each independently C₃-C₈-cycloalkyl or C₆-C₁₀-aryl, is reacted with a compound of formula III 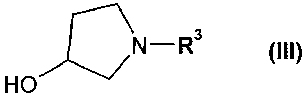 or an ester-forming derivative thereof, wherein R³ is C₁-C₈-alkyl to form a compound of formula IV 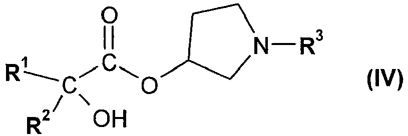 wherein R¹ and R² are each independently C₃-C₈-cycloalkyl or C₆-C₁₀-aryl and R³ is C₁-C₈-alkyl.

The reaction may be effected using known procedures for reacting hydroxy compounds or salts thereof (for example sodium salts) with carboxylic acids or ester-forming derivatives thereof such as acid halides or analogously as hereinafter described in the Examples. The reaction is conveniently carried out in an organic solvent, for example dimethylformamide (DMF) or toluene, in the presence of a coupling agent, for example 1,1'-carbonyldiimidazole (CDI), preferably in an inert atmosphere, e.g. under argon. Suitable reaction temperatures are from 0° C to 100° C, preferably from 30° C to 80° C, especially about 60° C.

When the coupling agent is 1,1'-carbonyldiimidazole, the active intermediate is a compound of formula IIa 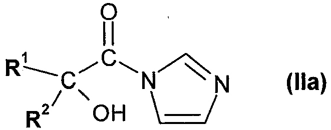 or a salt thereof wherein R¹ and R² are each independently C₃-C₈-cycloalkyl or C₆-C₁₀-aryl. In a preferred embodiment R¹ and R² are cyclopentyl and phenyl respectively so the compound of formula IIa is 2-cyclopentyl-2-hydroxy-1-imidazol-1-yl-2-phenyl-ethanone.

The compound of formula I may be purified by any suitable art known technique, for example recrystallisation, and/or any coarse particles may be removed by sieving.

The following suitable, preferred, more preferred or most preferred aspects of the invention may be incorporated independently, collectively or in any combination.

R¹ and R² are suitably each independently cyclopropyl, cyclohexyl or phenyl. Alternatively R¹ is suitably cyclopropyl and R² is suitably phenyl.

R³ is suitably methyl, ethyl, propyl, i-propyl, butyl, i-butyl or t-butyl. Alternatively R³ is suitably methyl.

R⁴ is suitably methyl, ethyl, propyl, i-propyl, butyl, i-butyl or t-butyl. Alternatively R⁴ is suitably methyl.

X is suitably chloro, bromo or iodo. Alternatively X is suitably bromo.

In a preferred embodiment R¹ and R² of the compounds of formulae II and IV are each independently C₅-C₆-cycloalkyl or phenyl; and R³ of the compounds of formulae III and IV is C₁-C₄-alkyl, especially methyl.

In another preferred embodiment R¹ of the compounds of formulae II and IV is C₅-C₆-cycloalkyl; R² of the compounds of formulae II and IV is phenyl; and R³ of the compounds of formulae III and IV is C₁-C₄-alkyl, especially methyl.

In yet another preferred embodiment R¹ of the compounds of formulae II and IV is cyclopentyl; R² of the compounds of formulae II and IV is phenyl; R³ of the compounds of formulae III and IV is methyl.

In the second step (b) of the process of the present invention, a compound of formula IV 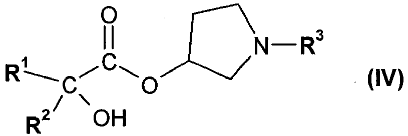 wherein R¹ and R² are each independently C₃-C₈-cycloalkyl or C₆-C₁₀-aryl and R³ is C₁-C₈-alkyl is reacted with a compound of formula V

X-R⁴ (V)

wherein R⁴ is C₁-C₈-alkyl and X is a leaving group, to form a compound of formula I in salt or zwitterionic form, wherein
R¹ and R² are each independently C₃-C₈-cycloalkyl or C₆-C₁₀-aryl; and
R³ and R⁴ are each independently C₁-C₈-alkyl.

The reaction may be effected using known procedures for reacting quinuclidinol esters with alkyl halides or analogously as hereinafter described in the Examples. The reaction is conveniently carried out in water or an organic solvent, for example acetonitrile, dimethylformamide (DMF), dimethylsulphoxide (DMSO), ethyl acetate or chloroform. The reaction is carried out at a temperature from about -10°C to about 120° C, conveniently from about -5°C to about 80° C, especially from about 0°C to about 60°C.

In a preferred embodiment the compound of formula V is methyl bromide. The compound is volatile (boiling point 4°C) so the reaction is initially carried out from about 0°C to about 20°C, then the reaction mixture is heated to about 60°C prior to crystallization. The crystallization is induced by cooling, i.e. lowering the temperature of the mixture, actively or passively. In a preferred embodiment the temperature of the mixture is lowered slowly, i.e. over several hours, using commercially available automated equipment. If desirable the reaction mixture is seeded to facilitate crystallisation. In a preferred embodiment the reaction mixture is cooled to about 50°C, then seeded, then cooled slowly to about 15°C.

The choice of solvent used in the alkylation reaction may influence the yield of particular stereoisomers of the desired compound significantly. Indeed it may be advantageous that step (b) is carried out in an organic solvent in which stereoisomers of the compound of formula I have differing solubility. For example when reacting cyclopentyl-hydroxy-phenyl-acetic acid 1-methyl-pyrrolidin-3-yl ester with methyl bromide in n-propanol to prepare glycopyrronium bromide the product that crystallises out of solution on cooling is enriched with (3S,2'R)- and (3R,2'S)-3-[(cyclopentyl-hydroxyphenyl-acetyl)-oxy]-1,1-dimethyl-pyrrolidinium bromide whereas (3R,2'R)- and (3S,2'S)-3-[(cyclo-pentyl-hydroxyphenyl-acetyl)-oxy]-1,1-dimethylpyrrolidinium bromide), which are much more soluble in n-propanol, tend to remain in the filtrate.

The following suitable, preferred, more preferred or most preferred aspects of the invention may be incorporated independently, collectively or in any combination.

R¹ and R² are suitably each independently cyclopentyl, cyclohexyl or phenyl. Alternatively R¹ is suitably cyclopropyl and R² is suitably phenyl.

R³ is suitably methyl, ethyl, propyl, i-propyl, butyl, i-butyl or t-butyl. Alternatively R³ is suitably methyl.

R⁴ is suitably methyl, ethyl, propyl, i-propyl, butyl, i-butyl or t-butyl. Alternatively R⁴ is suitably methyl.

X is suitably a halogen such as chloro, bromo or iodo. Alternatively X is suitably bromo.

X is suitably a sulfonic acid or phosphonic acid moiety such as mesylate, tosylate, benzenesulfonate or methyl methanephosphonate.

In a preferred embodiment R¹ and R² of the compound of formulae IV and I are each independently C₅-C₆-cycloalkyl or phenyl; R³ of the compounds of formulae IV and I is C₁-C₅-alkyl; and R⁴ of the compounds of formulae V and I is C₁-C₄-alkyl, especially methyl.

In another preferred embodiment R¹ of the compounds of formulae IV and I is C₅-C₆-cycloalkyl; R² of the compounds of formulae IV and I is phenyl; R³ of the compounds of formulae IV and I is C₁-C₄-alkyl, especially methyl; and R⁴ of the compounds of formula V and I is C₁-C₄-alkyl, especially methyl.

In yet another preferred embodiment R¹ of the compounds of formulae IV and I is cyclopentyl; R² of the compounds of formulae IV and I is phenyl; R³ of the compounds of formula IV and I is methyl; and R⁴ of the compounds of formula V and I is methyl so that the compound of formula I is glycopyrronium in salt or zwitterionic form.

The process of the present invention overcomes various problems identified with the process for preparing glycopyrrolate that is described in United States patent US 2,956,062. By removing the need to form the methyl ester from the acid as an extra step one shortens the process, improves yield and avoids having to employ a laborious transesterification method that is often difficult to control, difficult to optimise and involves using hazardous reagents such as sodium and sodium hydride and hazardous conditions such as forming hydrogen gas. It is convenient and time and cost-effective that the starting materials are commercially available acids and that the process can be carried out in one receptacle i.e. a simple, one pot process. These advantages make the process of the present invention significantly more suitable for large scale industrial manufacture than the process described in US 2,956,062. A preferred embodiment of the process of the present invention, insofar as the final product is glycopyrrolate, is summarized and compared with the process described in US 2,956,062 in the following scheme. In this scheme glycopyrrolate is prepared by the known process via stages 1, 2 and 3, whereas it is prepared by the process of the present invention via stages 1a and 3:

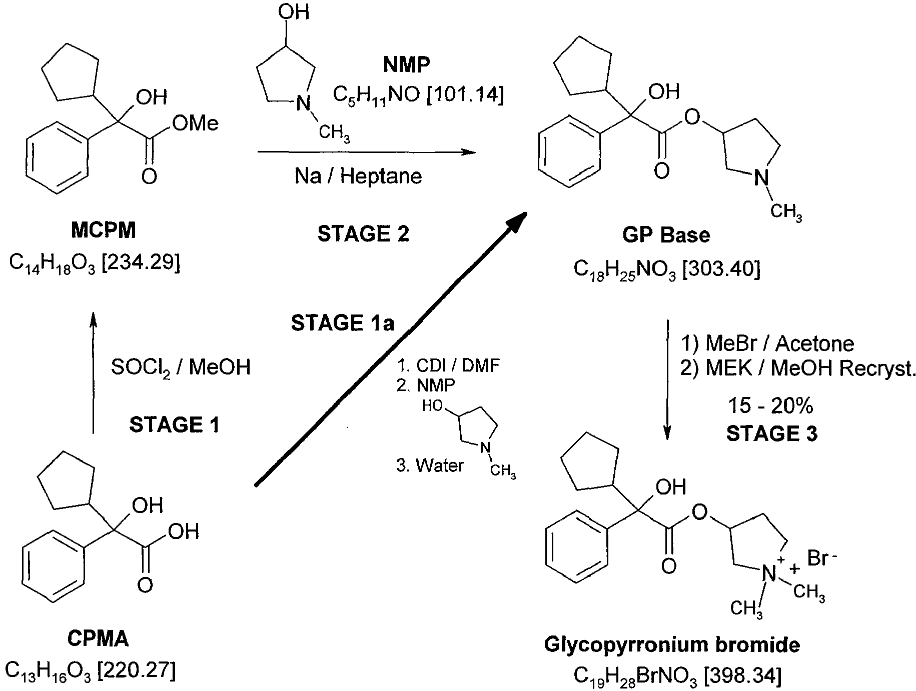

In a preferred embodiment R¹ and R² of the compound of formula II are cyclopentyl and phenyl respectively, R³ of the compound of formula III is methyl and R⁴ of the compound of formula V is methyl and the compound of formula I is a racemic mixture of (3S,2'R)- and (3R,2'S)-3-[(cyclopentyl-hydroxyphenylacetyl)-oxy]-1,1-dimethylpyrrolidinium bromide.

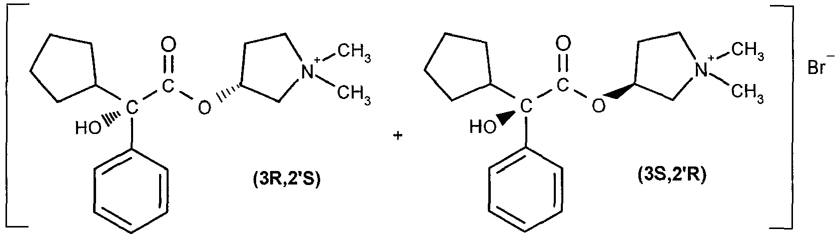

The process of the present invention minimizes variation in the relative proportions of these enantiomers of glycopyrrolate.

The present invention also provides a process for preparing inhalable dry powder formulations of a compound of formula I 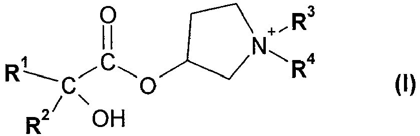 in salt or zwitterionic form, wherein R¹ and R² are each independently C₃-C₈-cycloalkyl or C₆-C₁₀-aryl; and R³ and R⁴ are each independently C₁-C₈-alkyl. That process comprises five steps (i)-(v).

Steps (i) and (ii) are identical to steps (a) and (b) of the aforementioned process for preparing a drug substance that comprises a compound of formula I in salt or zwitterionic form.

In the third step (iii) of the process for preparing an inhalable dry powder formulation, which step is optional, the drug substance comprising a compound of formula I in salt or zwitterionic form is purified by crystallisation. This step may be repeated as necessary until a desired purity is achieved. The drug substance may be sieved to remove any coarse particles.

In the fourth step (iv) of the process for preparing an inhalable dry powder formulation, the drug substance comprising a compound of formula I in salt or zwitterionic form, optionally purified according to step (iii), is micronised. This reduces the particle size of the drug substance so that it is suitable for administration by inhalation. The mass median aerodynamic diameter (MMAD) of these particles is preferably less than 10 microns (µm). Particles having aerodynamic diameters greater than about 10 µm are likely to impact the walls of the throat and generally do not reach the lung. Particles having aerodynamic diameters in the range of about 2 µm to about 5 µm will generally be deposited in the respiratory bronchioles whereas smaller particles having aerodynamic diameters in the range of about 0.05 µm to about 3 µm are likely to be deposited in the alveoli and to be absorbed into the bloodstream.

Micronising equipment is well known in the art and includes a variety of grinding and milling machinery, for example compressive-type mills such as mechanofusion mills, impact mills such as ball mills, homogenizers and micro fluidizers, and jet mills. Suitable micronising equipment includes low shear mixers such as a Turbula® powder blender and high-shear mixers such as a MiPro® powder blender.

In a preferred embodiment crystalline glycopyrrolate is jet milled in a Hosokawa Alpine® 100 AFG fluid bed opposed jet mill or a spiral jetmill is used, for example a Hosokawa Alpine® AS100 spiral mill). Other suitable jet milling equipment includes Hosokawa Alpine® AFG140, AFG200, AFG280 and AFG400 jet mills.

In the fifth step (v) of the process for preparing an inhalable dry powder formulation, carrier particles are admixed with the micronised crystalline drug substance to give the desired inhalable dry powder formulation. The carrier particles make the micronised drug substance less cohesive and improve its flowability. This makes the powder easier to handle downstream, for example when filling the dry powder formulation into capsules. The micronised drug substance particles tend to adhere to the surface of the carrier particles whilst stored in a dry powder inhaler device but are dispersed from the surfaces of the carrier particles on inhalation into the respiratory tract to give a fine suspension. The larger carrier particles are mostly deposited in the oropharyngeal cavity.

The carrier particles may be composed of any pharmacologically inert material or combination of materials which is acceptable for inhalation. They are suitably composed of one or more crystalline sugars including monosaccharides, disaccharides, polysaccharides and sugar alcohols such as arabinose, glucose, fructose, ribose, mannose, sucrose, trehalose, lactose, maltose, starches, dextran, mannitol or sorbitol. An especially preferred carrier is lactose, for example lactose monohydrate or anhydrous lactose.

Preferably substantially all (by weight) of the carrier particles have a diameter of 20 to 1000 µm, more preferably 50 to 500 µm, but especially 20 to 250 µm. The diameter of substantially all (by weight) of the carrier particles is suitably less than 355 µm. This provides good flow and entrainment characteristics and improved release of the active particles in the airways to increase deposition of the active particles in the lower lung. It will be understood that, throughout, the diameter of the particles referred to is the aerodynamic diameter of the particles.

When desirable, one or more force control agents such as magnesium stearate is included in dry powder formulations for inhalation. The force control agent leads to a general improvement in the inhalable fine particle fraction in dry powder glycopyrrolate formulations. It stabilizes the carrier materials and the drug substance by suppressing or slowing down undesirable morphological phase transitions. It also enhances the dosing efficiency of inhalable dry powder glycopyrrolate formulations by improving powder flowability.

Other suitable force control agents include amino acids such as leucine, phospholipids such as lecithin or fatty acid derivatives such calcium stearate. However magnesium stearate is especially preferred. It is preferably added in particularly small amounts, for example 0.1 to 5% by weight, more preferably 0.1 to 2% by weight, but especially about 0.25 to 1% by weight, based on the total formulation, of magnesium stearate.

The force control agent is preferably in particulate form but it may be added in liquid or solid form and for some materials, especially where it may not be easy to form particles of the material and/or where those particles should be especially small, it may be preferred to add the material in a liquid, for example as a suspension or a solution.

The dry powder may be contained as unit doses in capsules of, for example, gelatin or plastic, or in blisters (e.g. of aluminium or plastic), for use in a dry powder inhalation device, which may be a single dose or multiple dose device. Preferably the total weight of powder per capsule is from 5 mg to 50 mg. Alternatively, the dry powder may be contained in a reservoir in a multi-dose dry powder inhalation (MDDPI) device adapted to deliver, for example, 3-25 mg of dry powder per actuation. A suitable device for delivery of dry powder in encapsulated form is described in US 3,991,761 or WO 05/113042, while suitable MDDPI devices include those described in WO 97/20589 and WO 97/30743.

The invention is illustrated by the following Examples.

### EXAMPLE

### Example 1

### Preparation of (3S,2'R)- and (3R,2'S)-3-[(cyclopentyl-hydroxyphenylacetyl)-oxy]-1,1-dimethylpyrrolidinium bromide

30 g of cyclopentyl mandelic acid, dissolved in 135 g dimethylformamide (DMF), were treated with 27 g carbonyldiimidazole at 18°C (in portions) to form the "active amide". After the addition of 16.9 g of 1-methyl-pyrrolidin-3-ol, the mixture was heated to 60°C within 1 hour and stirred for 18 hours at this temperature. After checking for complete conversion, the mixture was cooled and 200 g water was added. The mixture was extracted with 200 g toluene and the extract was washed with water three times. The organic phase was concentrated to obtain cyclopentyl-hydroxy-phenyl-acetic acid 1-methyl-pyrrolidin-3-yl ester as an about 50% solution in toluene, ready to use for the next step.

This solution was diluted with 120 g of n-propanol and cooled to 0°C. 16.8 g methyl bromide was introduced and the mixture was stirred for 2 hours and then gradually heated to 60°C to evaporate the excess methyl bromide into a scrubber. The mixture was then cooled to 50°C and seed crystals were added to facilitate crystallisation. The temperature was then slowly reduced over 18 hours to 15°C. The solid was then isolated by filtration to obtain 22.7 g after drying. It was composed mainly of one pair of enantiomers, a racemic mixture of (3S,2'R)- and (3R,2'S)-3-[(cyclopentyl-hydroxyphenylacetyl)-oxy]-1,1-dimethylpyrrolidinium bromide, with a purity greater than 90% (by HPLC). The other pair of diastereoisomers ((3R,2'R)- and (3S,2'S)-3-[(cyclopentyl-hydroxyphenyl-acetyl)-oxyl-1,1-dimethylpyrrolidinium bromide) remains mainly in the filtrate as those compounds are significantly more soluble in n-propanol than the other stereoisomers.

The solid obtained is further recrystallised in n-propanol (1:10 wt) to give pure (3S,2'R)- and (3R,2'S)-3-[(cyclopentyl-hydroxyphenylacetyl)-oxy]-1,1-dimethylpyrrolidinium bromide i.e. purity > 99.9% as determined by high performance liquid chromatography (HPLC).

This process is summarised in the following reaction scheme:

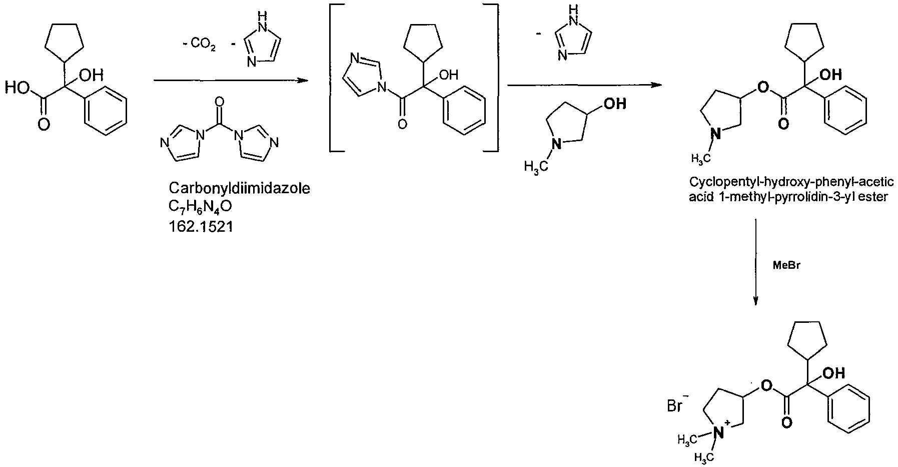

### Reference Example 2

### Preparation of cyclopentyl-hydroxy-phenyl-acetic acid 1-methyl-pyrrolidin-3yl-ester in toluene

1 g of cyclopentyl mandelic acid was suspended in 4.7 g of toluene and 1.5 g of carbonyldiimidazole were added as a solid. After 30 minutes 0.69 g of 1-methyl-pyrrolidin-3-ol and 20 mg of sodium tert-butylate were added. The mixture was stirred at room temperature for 18 hours then water was added. After stirring the phases were separated and the organic phase was washed with water twice and evaporated to obtain an approximately 50% solution of cyclopentyl-hydroxy-phenyl-acetic acid 1-methyl-pyrrolidin-3yl-ester in toluene.

### Example 3

### Preparation of 2-cyclopentyl-2-hydroxy-1-imidazol-1-yl-2-phenyl-ethanone, the active intermediate

The imidazolidyl derivative of cyclopentylmandelic acid was prepared and isolated as a solid by the following method:

10 g of cyclopentylmandelic acid were suspended in 30 ml of acetonitrile and the mixture was cooled to 0°C. 10.3 g of carbonyldiimidazole were added as a solid and the mixture was warmed to room temperature for 2 hours. Carbon dioxide evolved as a gas as a precipitate formed. The mixture was then cooled to 5°C and the solid was filtered, washed with acetonitrile and dried in vacuum at 40°C to obtain 7.3 g of pure 2-cyclopentyl-2-hydroxy-1-imidazol-1-yl-2-phenyl-ethanone.

This process is summarised in the following reaction scheme:

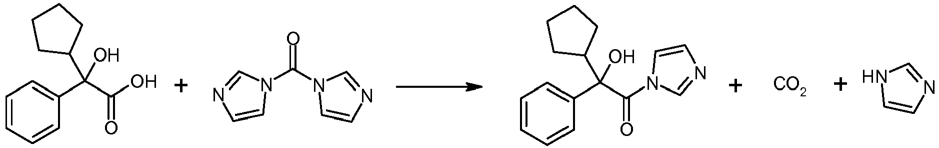

High resolution MS-spectroscopy revealed the molecular formula of the compound (as M+H) to be C₁₆H₁₉O₂N₂ with an exact mass of 271.14414 (0.14575ppm deviation from the calculated value).
¹H-NMR-spectroscopy (600MHz, DMSO-d₆): 1.03-1.07 (m, 1H), 1.25-1.30 (m, 1H), 1.35-1.40 (m, 1H), 1.40-1.50 (m, 1H), 1.53-1.56 (m, 2H), 1-60-1.67 (m, 1H), 1.75-1.84 (m, 1H), 1.03 - 1.85 (8H, 8 secondary CH₂-protons in the cyclopentylring, H-C11, H-C12, H-C13, H-C14); 2.7-2.9 (m, 1H, H-C10); 6.76 (1H, H-C5); 6.91 (1H, H-C4); 7.29 (1H, H-C18); 7.39 (2H, H-C17, H-C19); 7.49 (2H, H-C16, H-C20); 7.65 (1H, H-C2).

The compound was characterised by IR-spectroscopy (measured as a solid film on a BRUKER TENSOR 27 FT-IR spectrometer over a wave number range of 4000-600 cm⁻¹ with a resolution of 4 cm⁻¹). An assignment of the most important bands is given below:

| Wavenumber (cm⁻¹) | Assignments |
|---|---|
| 3300 ∼ 2500 | O-H stretching |
| 3167, 3151, 3120 | Imidazole CH stretching |
| 2956, 2868 | Cyclopentyl CH stretching |
| 1727 | C=O stretching |
| 1600, 1538, 1469 | Aromatic rings stretching |
| 735 | Mono-subst. benzene CH o.o.p. bending |
| 704 | Mono-subst. benzene ring o.o.p. bending |

## Claims

1. A process for preparing a compound of formula I 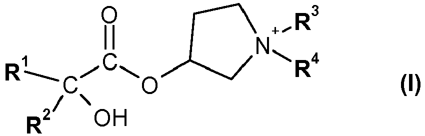 in salt or zwitterionic form, wherein
R¹ and R² are each independently C₃-C₈-cycloalkyl or C₆-C₁₀-aryl; and
R³ and R⁴ are each independently C₁-C₈-alkyl;
the process comprising the steps of:
(a) reacting a compound of formula II 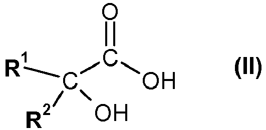 or a salt thereof wherein R¹ and R² are each independently C₃-C₈-cycloalkyl or C₆-C₁₀-aryl, with a compound of formula III 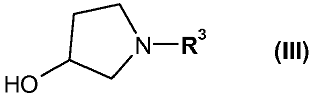 or an ester-forming derivative thereof, wherein R³ is C₁-C₈-alkyl to form a compound of formula IV 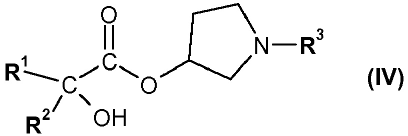
wherein R¹ and R² are each independently C₃-C₈-cycloalkyl or C₆-C₁₀-aryl and R³ is C₁-C₈-alkyl, and
wherein the reaction is carried out in the absence of sodium and sodium hydride; and
(b) reacting a compound of formula IV wherein R¹ and R² are each independently C₃-C₈-cycloalkyl or C₆-C₁₀-aryl and R³ is C₁-C₈-alkyl with a compound of formula V
X-R⁴ (V)
wherein R⁴ is C₁-C₈-alkyl and X is a leaving group, to form a compound of formula I in salt or zwitterionic form, wherein
R¹ and R² are each independently C₃-C₈-cycloalkyl or C₆-C₁₀-aryl; and
R³ and R⁴ are each independently C₁-C₈-alkyl.

2. A process according to claim 1 wherein
R¹ and R² of the compound of formula II and IV are each independently C₅-C₆-cycloalkyl or phenyl; and
R³ of the compounds of formulae III and IV is C₁-C₄-alkyl.

3. A process according to claim 1 or 2 wherein
R¹ and R² of the compound of formulae IV and I are each independently C₅-C₆-cycloalkyl or phenyl;
R³ of the compounds of formulae IV and I is C₁-C₄-alkyl; and
R⁴ of the compounds of formulae V and I is C₁-C₄-alkyl.

4. A process according to any preceding claim wherein
R¹ of the compound of formulae II and IV is cyclopentyl;
R² of the compound of formulae II and IV is phenyl;
R³ of the compound of formula III and IV is methyl; and
R⁴ of the compound of formula V is methyl so that the compound of formula I is glycopyrronium in salt or zwitterionic form.

5. A process according to claim 4 wherein the compound of formula I is a racemic mixture of (3S,2'R)- and (3R,2'S)-3-[(cyclopentyl-hydroxyphenylacetyl)-oxy]-1,1-dimethylpyrrolidinium bromide.

6. A process according to any preceding claim wherein step (a) is carried out in the presence of a coupling agent.

7. A process according to claim 6 wherein the coupling agent is carbonyldiimidazole.

8. A process according to any preceding claim wherein step (b) is carried out in an organic solvent in which stereoisomers of the compound of formula I have differing solubility.

9. A process for preparing an inhalable dry powder formulation of a compound of formula I 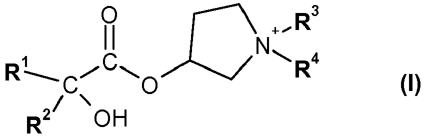 in salt or zwitterionic form, wherein
R¹ and R² are each independently C₃-C₈-cycloalkyl or C₆-C₁₀-aryl; and
R³ and R⁴ are each independently C₁-C₈-alkyl;
the process comprising the steps of:
(i) reacting a compound of formula II 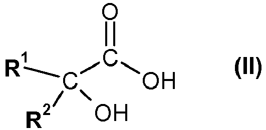 or a salt thereof wherein R¹ and R² are each independently C₃-C₈-cycloalkyl or C₆-C₁₀-aryl, with a compound of formula III 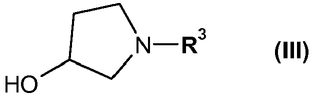 or an ester-forming derivative thereof, wherein R³ is C₁-C₈-alkyl to form a compound of formula IV 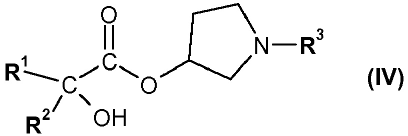
wherein R¹ and R² are each independently C₃-C₈-cycloalkyl or C₆-C₁₀-aryl and R³ is C₁-C₈-alkyl,
wherein the reaction is carried out in the absence of sodium and sodium hydride;
(ii) reacting a compound of formula IV wherein R¹ and R² are each independently C₃-C₈-cycloalkyl or C₆-C₁₀-aryl and R³ is C₁-C₈-alkyl with a compound of formula V
X-R⁴ (V)
wherein R⁴ is C₁-C₈-alkyl and X is a leaving group, to form a drug substance that comprises a compound of formula I in salt or zwitterionic form, wherein R¹ and R² are each independently C₃-C₈-cycloalkyl or C₆-C₁₀-aryl; and
R³ and R⁴ are each independently C₁-C₈-alkyl;
(iii) optionally purifying the drug substance by crystallisation to provide a purified drug substance;
(iv) micronising the drug substance; and
(v) admixing carrier particles to give the inhalable dry powder.

10. A process according to claim 9 wherein in step (iv) the drug substance is micronised together with a force control agent.

11. A process according to claim 10 wherein the force control agent is magnesium stearate.

## Patentansprüche

1. Verfahren zur Herstellung einer Verbindung der Formel I: 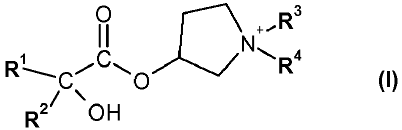 in einer salzartigen oder zwitterionischen Form, wobei
R¹ und R² jeweils auf unabhängige Weise für C₃-C₈-Cycloalkyl oder C₆-C₁₀-Aryl stehen; und
R³ und R⁴ jeweils auf unabhängige Weise für C₁-C₈-Alkyl stehen;
wobei das Verfahren die folgenden Schritte umfasst:
(a) Umsetzen einer Verbindung der Formel II 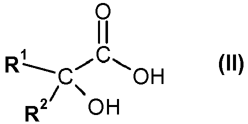 oder eines Salzes derselben, wobei R¹ und R² jeweils auf unabhängige Weise für C₃-C₈-Cycloalkyl oder C₆-C₁₀-Aryl stehen, mit einer Verbindung der Formel III 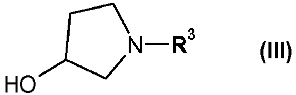 oder einem Esterderivat derselben, wobei R³ für C₁-C₈-Alkyl steht, um eine Verbindung der Formel IV zu bilden 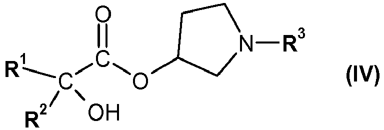
wobei R¹ und R² jeweils auf unabhängige Weise für C₃-C₈-Cycloalkyl oder C₆-C₁₀-Aryl stehen und R³ für C₁-C₈-Alkyl steht, und
wobei die Umsetzung in Abwesenheit von Natrium und Natriumhydrid durchgeführt wird; und
(b) Umsetzen einer Verbindung der Formel IV, wobei R¹ und R² jeweils auf unabhängige Weise für C₃-C₈-Cycloalkyl oder C₆-C₁₀-Aryl stehen und R³ für C₁-C₈-Alkyl steht, mit einer Verbindung der Formel V
X-R⁴ (V)
wobei R⁴ für C₁-C₈-Alkyl steht und X für eine Abgangsgruppe steht, um eine Verbindung der Formel I in einer salzartigen oder zwitterionischen Form zu bilden, wobei
R¹ und R² jeweils auf unabhängige Weise für C₃-C₈-Cycloalkyl oder C₆-C₁₀-Aryl stehen; und
R³ und R⁴ jeweils auf unabhängige Weise für C₁-C₈-Alkyl stehen.

2. Verfahren nach Anspruch 1, wobei
R¹ und R² der Verbindung der Formel II und IV jeweils auf unabhängige Weise für C₅-C₆-Cycloalkyl oder Phenyl stehen; und
R³ der Verbindungen der Formeln III und IV für C₁-C₄-Alkyl steht.

3. Verfahren nach Anspruch 1 oder 2, wobei
R¹ und R² der Verbindung der Formeln IV und I jeweils auf unabhängige Weise für C₅-C₆-Cycloalkyl oder Phenyl stehen;
R³ der Verbindungen der Formeln IV und I für C₁-C₄-Alkyl steht, und
R⁴ der Verbindungen der Formeln V und I für C₁-C₄-Alkyl steht.

4. Verfahren nach einem beliebigen der vorhergehenden Ansprüche, wobei
R¹ der Verbindung der Formeln II und IV gleich Cyclopentyl ist;
R² der Verbindung der Formeln II und IV gleich Phenyl ist;
R³ der Verbindung der Formel III und IV gleich Methyl ist; und
R⁴ der Verbindung der Formel V gleich Methyl ist, sodass die Verbindung der Formel I gleich Glycopyrronium in salzartiger oder zwitterionischer Form ist.

5. Verfahren nach Anspruch 4, wobei die Verbindung der Formel I ein Racemat von (3S,2'R)- und (3R,2'S)-3-[(Cyclopentylhydroxyphenylacetyl)-oxy]-1,1-dimethylpyrrolidiniumbromid ist.

6. Verfahren nach einem beliebigen der vorhergehenden Ansprüche, wobei Schritt (a) in Gegenwart eines Kupplungsmittels durchgeführt wird.

7. Verfahren nach Anspruch 6, wobei es sich bei dem Kupplungsmittel um Carbonyldiimidazol handelt.

8. Verfahren nach einem beliebigen der vorhergehenden Ansprüche, wobei Schritt (b) in einem organischen Lösungsmittel durchgeführt wird, in welchem die Stereoisomere der Verbindung der Formel I unterschiedliche Löslichkeiten aufweisen.

9. Verfahren zur Herstellung einer inhalierbaren Trockenpulverformulierung einer Verbindung der Formel I 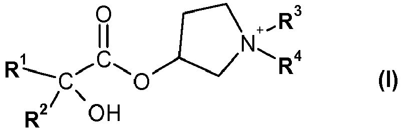 in einer salzartigen oder zwitterionischen Form, wobei
R¹ und R² jeweils auf unabhängige Weise für C₃-C₈-Cycloalkyl oder C₆-C₁₀-Aryl stehen; und
R³ und R⁴ jeweils auf unabhängige Weise für C₁-C₈-Alkyl stehen;
wobei das Verfahren die folgenden Schritte umfasst:
(i) Umsetzen einer Verbindung der Formel II 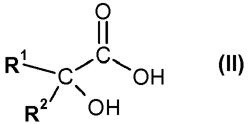 oder eines Salzes derselben, wobei R¹ und R² jeweils auf unabhängige Weise für C₃-C₈-Cycloalkyl oder C₆-C₁₀-Aryl stehen, mit einer Verbindung der Formel III 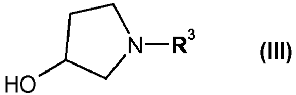 oder einem Esterderivat derselben, wobei R³ für C₁-C₈-Alkyl steht, um eine Verbindung der Formel IV zu bilden 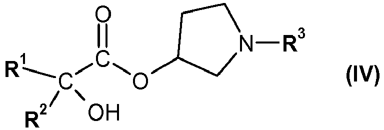
wobei R¹ und R² jeweils auf unabhängige Weise für C₃-C₈-Cycloalkyl oder C₆-C₁₀-Aryl stehen und R³ für C₁-C₈-Alkyl steht,
wobei die Umsetzung in Abwesenheit von Natrium und Natriumhydrid durchgeführt wird;
(ii) Umsetzen einer Verbindung der Formel IV, wobei R¹ und R² jeweils auf unabhängige Weise für C₃-C₈-Cycloalkyl oder C₆-C₁₀-Aryl stehen und R³ für C₁-C₈-Alkyl steht, mit einer Verbindung der Formel V
X-R⁴ (V)
wobei R⁴ für C₁-C₈-Alkyl steht und X für eine Abgangsgruppe steht, um einen Arzneistoff zu bilden, der eine Verbindung der Formel I in einer salzartigen oder zwitterionischen Form umfasst, wobei R¹ und R² jeweils auf unabhängige Weise für C₃-C₈-Cycloalkyl oder C₆-C₁₀-Aryl stehen; und R³ und R⁴ jeweils auf unabhängige Weise für C₁-C₈-Alkyl stehen;
(iii) möglicherweise Aufreinigen des Arzneistoffs durch Kristallisation, um einen aufgereinigten Arzneistoff bereitzustellen;
(iv) Mikrovermahlen des Arzneistoffs; und
(v) Beimischen von Trägerstoffpartikeln, um ein inhalierbares Trockenpulver zu erhalten.

10. Verfahren nach Anspruch 9, wobei der Arzneistoff in Schritt (iv) zusammen mit einem Pulverdispersionsmittel mikrovermahlen wird.

11. Verfahren nach Anspruch 10, wobei es sich bei dem Pulverdispersionsmittel um Magnesiumstearat handelt.

## Revendications

1. Procédé de synthèse d'un composé de formule I 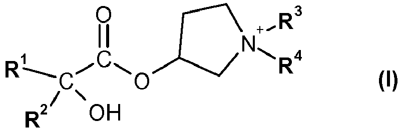 sous forme saline ou zwitterionique, où
chacun des radicaux R¹ et R² représente indépendamment un groupement cycloalkyle en C₃-C₈ ou aryle en C₆-C₁₀ ; et
chacun des radicaux R³ et R⁴ représente indépendamment un groupement alkyle en C₁-C₈ ;
le procédé comprenant les étapes suivantes :
(a) réaction d'un composé de formule II 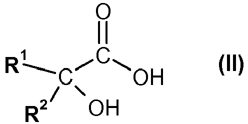 ou d'un sel de celui-ci, où chacun des radicaux R¹ et R² représente indépendamment un groupement cycloalkyle en C₃-C₈ ou aryle en C₆-C₁₀, avec un composé de formule III 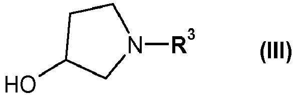 ou un dérivé formant un ester de celui-ci, où R³ représente un groupement alkyle en C₁-C₈, pour former un composé de formule IV 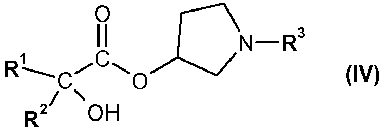
où chacun des radicaux R¹ et R² représente indépendamment un groupement cycloalkyle en C₃-C₈ ou aryle en C₆-C₁₀, et R³ représente un groupement alkyle en C₁-C₈, et
où la réaction est mise en oeuvre en l'absence de sodium et d'hydrure de sodium ; et
(b) réaction d'un composé de formule IV où chacun des radicaux R¹ et R² représente indépendamment un groupement cycloalkyle en C₃-C₈ ou aryle en C₆-C₁₀ et R³ représente un groupement alkyle en C₁-C₈ avec un composé de formule V
X-R⁴ (V)
où R⁴ représente un groupement alkyle en C₁-C₈ et X représente un groupement partant, pour former un composé de formule I sous forme saline ou zwitterionique, où
chacun des radicaux R¹ et R² représente indépendamment un groupement cycloalkyle en C₃-C₈ ou aryle en C₆-C₁₀ ; et
chacun des radicaux R³ et R⁴ représente indépendamment un groupement alkyle en C₁-C₈.

2. Procédé selon la revendication 1, où
chacun des radicaux R¹ et R² des composés de formule II et IV représente indépendamment un groupement cycloalkyle en C₅-C₆ ou phényle ; et
R³ dans les composés de formule III et IV représente un groupement alkyle en C₁-C₄.

3. Procédé selon la revendication 1 ou 2, où
chacun des radicaux R¹ et R² des composés de formule IV et I représente indépendamment un groupement cycloalkyle en C₅-C₆ ou phényle ;
R³ dans les composés de formule IV et I représente un groupement alkyle en C₁-C₄ ; et
R⁴ dans les composés de formule V et I représente un groupement alkyle en C₁-C₄.

4. Procédé selon l'une quelconque des revendications précédentes, où
R¹ dans les composés de formule II et IV représente un groupement cyclopentyle ;
R² dans les composés de formule II et IV représente un groupement phényle ;
R³ dans les composés de formule III et IV représente un groupement méthyle ; et
R⁴ dans le composé de formule V représente un groupement méthyle, de sorte que le composé de formule I soit le glycopyrronium sous forme saline ou zwitterionique.

5. Procédé selon la revendication 4, où le composé de formule I est un mélange racémique de bromure de (3S,2'R) et de (3R,2'S)-3-[(cyclopentyl-hydroxyphénylacétyl)-oxy]-1,1-diméthylpyrrolidinium.

6. Procédé selon l'une quelconque des revendications précédentes, où l'étape (a) est mise en oeuvre en présence d'un agent de couplage.

7. Procédé selon la revendication 6, où l'agent de couplage est le carbonyldiimidazole.

8. Procédé selon l'une quelconque des revendications précédentes, où l'étape (b) est mise en oeuvre dans un solvant organique dans lequel les stéréoisomères du composé de formule I ont des solubilités différentes.

9. Procédé d'élaboration d'une formule de poudre sèche pour inhalation d'un composé de formule I 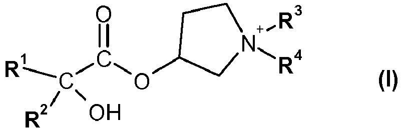 sous forme saline ou zwitterionique, où
chacun des radicaux R¹ et R² représente indépendamment un groupement cycloalkyle en C₃-C₈ ou aryle en C₆-C₁₀ ; et
chacun des radicaux R³ et R⁴ représente indépendamment un groupement alkyle en C₁-C₈ ;
le procédé comprenant les étapes suivantes :
(i) réaction d'un composé de formule II 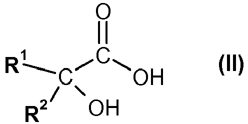 ou d'un sel de celui-ci, où chacun des radicaux R¹ et R² représente indépendamment un groupement cycloalkyle en C₃-C₈ ou aryle en C₆-C₁₀, avec un composé de formule III 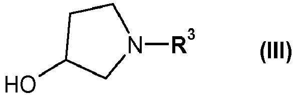 ou un dérivé formant un ester de celui-ci, où R³ représente un groupement alkyle en C₁-C₈, pour former un composé de formule IV 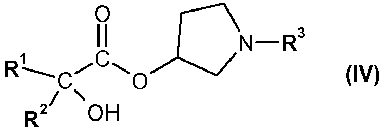
où chacun des radicaux R¹ et R² représente indépendamment un groupement cycloalkyle en C₃-C₈ ou aryle en C₆-C₁₀, et R³ représente un groupement alkyle en C₁-C₈,
où la réaction est mise en oeuvre en l'absence de sodium et d'hydrure de sodium ;
(ii) réaction d'un composé de formule IV où chacun des radicaux R¹ et R² représente indépendamment un groupement cycloalkyle en C₃-C₈ ou aryle en C₆-C₁₀ et R³ représente un groupement alkyle en C₁-C₈ avec un composé de formule V
X-R⁴ (V)
où R⁴ représente un groupement alkyle en C₁-C₈ et X représente un groupement partant, pour former une substance médicamenteuse comprenant un composé de formule I sous forme saline ou zwitterionique, où
chacun des radicaux R¹ et R² représente indépendamment un groupement cycloalkyle en C₃-C₈ ou aryle en C₆-C₁₀ ; et
chacun des radicaux R³ et R⁴ représente indépendamment un groupement alkyle en C₁-C₈ ;
(iii) purification éventuelle de la substance médicamenteuse par cristallisation pour former une substance médicamenteuse purifiée ;
(iv) micronisation de la substance médicamenteuse ; et
(v) mélangeage de particules vectrices pour obtenir la poudre sèche pour inhalation.

10. Procédé selon la revendication 9, où dans l'étape (iv), la substance médicamenteuse est micronisée avec un agent de contrôle de force.

11. Procédé selon la revendication 10, où l'agent de contrôle de force est le stéarate de magnésium.
